# EUROPEAN PATENT SPECIFICATION

(11) **EP 1 958 522 B1**
(45) Date of publication and mention of the grant of the patent: **05.09.2012**
(21) Application number: 06841721.1
(22) Date of filing: 30.11.2006
(51) Int. Cl.: A23L 1/30, A23L 1/308, A61K 31/201, A61K 31/575, A61K 31/716, A61P 9/10

(54) **FUNCTIONAL FOOD HAVING POSITIVE EFFECTS IN THE PREVENTION OF CARDIOVASCULAR DISEASES**
FUNCTIONAL FOOD MIT POSITIVEN AUSWIRKUNGEN BEI DER VORBEUGUNG VON HERZ-KREISLAUF-KRANKHEITEN
ALIMENT FONCTIONNEL A EFFETS POSITIFS DANS LA PREVENTION DE MALADIES CARDIOVASCULAIRES

(30) Priority: 30.11.2005 ES 200502963
(43) Date of publication of application: 20.08.2008
(73) Proprietor: La Morella Nuts, S.A., 43206 Reus (Tarragona) (ES)
(72) Inventor: RAMIREZ MARCO, Bartolome, E-43007 Tarragona (ES); ANGLES LLAURADO, Maria, Neus, E-43204 Reus (ES); REGUANT MIRANDA, Jordi, E-43204 Reus (ES); GODAS BONFILL, Gemma, E-08020 Barcelona (ES); SOLA ALBERICH, Rosa, E-43201 Reus (Tarragona) (ES)
(74) Representative: Carpintero Lopez, Francisco
(86) International application number: PCT/ES2006/000669
(87) International publication number: WO 2007/063158

(56) References cited:
- EP-A1- 1 518 464
- WO-A1-99/45797
- WO-A2-01/78529
- US-A1- 2004 131 657
- US-A1- 2005 100 619
- STEINBERG ET AL: "Cocoa and chocolate flavonoids: Implications for cardiovascular health" JOURNAL OF THE AMERICAN DIETETIC ASSOCIATION, THE ASSOCIATION, CHICAGO, IL, US, vol. 103, no. 2, 1 February 2003 (2003-02-01), pages 215-223, XP005613685 ISSN: 0002-8223
- GYLLING H ET AL: "PLANT STEROLS IN NUTRITION" SCANDINAVIAN JOURNAL OF NUTRITION, NAERINGSFORKNING, STOCKHOLM, SE, vol. 44, 1 January 2000 (2000-01-01), pages 155-157, XP002937208 ISSN: 1102-6480

## Description

### Field of the Invention

The present invention belongs to the field of functional foods. More particularly, it relates to a new food, or functional formulation, based on a mixture of food raw materials providing a positive effect on vascular diseases.

### Background of the Invention

According to a definition that the European Union and the International Life Science Institute Europe have agreed on, a food can be considered functional if it is proven to beneficially affect one or more functions of the organism beyond nutritional effects in the sense that it is relevant for an optimal health condition or for reducing the risk of suffering a disease (Diplock AT, Aggett PJ, Ashwell M. et al. Scientific concepts of functional foods in Europe: consensus document. Br.J. Nutr. 1999; 81 (suppl): S1-S27*).*

In recent years a considerable number of patents have been published which relate to supplements, ingredients or matrices which in turn incorporate a raw material or materials rich in bioactive components. One or several functionalities is associated to these bioactive compounds. Specifically, there are a number of patent documents relating to functional foods and/or products that are beneficial for the cardiovascular system: (US6610320), (US6747059), (W09945797), (W09809533), (WO0033669), (CN1399915), (W020044131657), (W02004052380), (US2004067921), (DE10233342), (WO02082929), (WO0224212), (EP1177729), (US6136367), (CA1239587), (EP0550060), (US5545414), (US2003/0134028), (US6787151), (US6251400), (WO98/434998), (US6210686), (WO02/060465)

Very recent scientific studies show how it is possible to achieve the same beneficial physiological effects by designing a diet incorporating a group of especially healthy ingredients as taking some mixtures of drugs defined for the same purpose *(*Franco OH, Bonneux L, de Laet C, Peeters A, Steyerberg EW, Mackenbach JP. The Polymeal: a more natural, safer, and probably tastier (than the Polypill) strategy to reduce cardiovascular disease by more than 75%. BMJ 2004; 329:1447-1450*).* This study proves that it is possible to reduce the risk of developing cardiovascular disease by more than 75% by introducing 7 types of ingredients in the diet, these ingredients including nuts, specifically almonds, dark chocolate, wine, fish, fruit, vegetables and garlic. All these ingredients incorporated in the diet in suitable proportions ("Polymeal") cause an effect that is comparable to the effect of mixing six specific drugs to reduce the risk of cardiovascular disease ("Polypill") (Wald NL, Law MR. A strategy to reduce cardiovascular disease by more than 80%. BMJ, 2003; 326:1419-23*).* The same objectives are thus achieved in a more organoleptically attractive manner while at the same time preventing possible side effects connected to the use of some medicinal products. This concept reinforces the idea of the search for balance and synergy among the active ingredients present in certain food groups.

In addition there are a number of studies proving the greater effectiveness of bioactive compounds within matrices of origin than when administered isolated therefrom (Liu RH. Health benefits of fruit and vegetables are from additive and synergistic combinations of phytochemicals. Am J Clin Nutr, 2003; 78 (suppl):517S-20S). Synergistic effects can thus occur among the components of the original raw materials, an effect which would probably not be seen with the use of isolated and purified bioactive components.

A number of recent scientific intervention and epidemiological studies show the convenience of incorporating nuts and, on the other hand, products derived from cocoa as well, such as chocolate, into the diet. Both food families are rich in bioactive components, beneficial in the development of certain physiological functions of the organism. Among the different health benefits are those relating to the prevention and reduction of the risk of suffering cardiovascular diseases, one of the diseases causing one of the highest mortality rates today *(*Fraser GE, Sabaté J, Beeson WL, Strahan TM. A possible protective effect of nut consumption on risk of coronary heart disease: the Adventist Health Study. Arch Intern Med 1992, 152:1416-24*); (*Fraser GE, Lindsted KD, Beeson WL. Effects of risk factor values on lifetime risk of and age at first coronary event. Am J Epidemiol 1995, 142: 746-758*); (*Fraser GE, Shavlik DJ. Risk factors for all-cause and coronary heart disease mortality in the oldest-old. Arch Intern Med 1997, 157:2249-2258*); (Hu FB,* Stampfer MJ, Manson JE, Rimm EB, Colditz GA, Rosner BA, Speizer FE, Hennekens CH, Willett WC. Frequent nut consumption and risk of coronary heart disease in women: prospective cohort study. BMJ 1998, 317:1341-5*); (*Kushi LH, Folsom AR, Prineas RJ, Mink PJ, Wu Y, Bostick RM. Dietary antioxidant vitamins and death from coronary disease in postmenopausal women. N Engl J Med 1996: 334: 1156-62*); (*Lavedrine F, Zmirou D, Ravel A, Balducci F, Alary J. Blood cholesterol and walnut consumption: a cross-sectional survey in France. Prev Med 28: 33-9, 1999*); (*Sabaté J. Nut consumption, vegetarian diets, ischemic heart disease risk, and all-cause mortality: evidence from epidemiologic studies. Am J Clin Nutr 1999; 70: 500S-3S*); (*Fraser GE. Nut consumption, lipids and risk of a coronary event. Clin Cardiol 1999; 22 (Supp III): III-11-15*); (*Solà R, Cabré P, Masana L. Importancia de los frutos secos. Revisión y aportaciones españolas a su estudio. Clin Invest Arteriosclerosis 2000; 12: 27-30*); (*Kris-Etherton PM, Zhao G, Biskoski AE, Stacie M, Coval BS, Etherton TD. Effects of nuts on coronary heart disease risk. Nutr Reviews 2001; 59: 103-111*).* Today it is known that patients presenting cardiovascular risk factors and a history of strokes further have an increased risk of suffering vascular dementia, as well as Alzheimer's disease. In particular, the apo B/apo A-I ratio is a marker of the proatherogenic and antiatherogenic particles in plasma. This ratio is used as a predictive marker of possible vascular problems; it has specifically been related to the risk of suffering myocardial infarction and is also connected to the risk of suffering a stroke *(*Yusuf, S. et al., The Interheart study: Case-control study. Lancet 2004; 364: 937-952*;* Walldius, G. et al. Stroke mortality and the apo B/apo A-I ratio: results of the Amoris prospective study. J Internal Med 2006; 259: 259-266*).*

In the case of neurodegenerative diseases, recent literature (Michikawa M. Cholesterol paradox: Is high total or low HDL cholesterol level a risk for Alzheimer Disease? Journal of Neuroscience Research 2003; 72:141-146*); (*Kado DM, Karlamanga AS, Huang MH, Troen A, Rowe JW, Selhub J, Seeman T. Homocysteine versus the vitamins folate, B6, and B12 as predictors of cognitive function and decline in older high-functioning adults: MacArthur Studies of Successful Aging*); (*Mattson MP, Duan W, Wan R, Guo Z. Prophylactic Activation of Neuroprotective Stress Response Pathways by Dietary and Behavioral Manipulations. The Journal of the American Society for Experimental Neuro Therapeutics 2004; 1:111-116) show that a good regulation of plasma lipid profiles, of homocysteine levels or the bioavailability of active ingredients with antioxidant properties play a preventive role, or a role of reducing, the risk of developing these diseases. In this sense many of the active ingredients that are beneficial in vascular diseases would coincide in this double function.

This type of food has a wide range of bioactive components (*Fraser, 1999; Kris-Etherton et al., 2001); (USDA U.S. Department of Agriculture Research Service Nutrient Database for Standard Reference, 1998); (*Jenkins DJA, Kendall CWC, Axelsen M, Augustin LSA, Vuksan V. Viscous and nonviscous fibers, nonabsorbable and low glycaemic index carbohydrates, blood lipids and coronary heart disease. Curr Opin Lipidol 2000, 11:49-56*); (*Brown A, Hu F. Dietary modulation of endothelial function: implications for cardiovascular disease. Am J Clin Nutr 2001; 73: 673-86*); (*Craig W, Beck L. Phytochemicals: health protective effects. C J Diet P Resch 1999; 12: 729-742; Mazur W. Phytoestrogen content in foods. B Clin Endo Metab 1998; 12: 729-742); (Van- der-Schouw YT. Phyto-oestrogens and cardiovascular disease risk. Nutr. Metab. Cardio Disease 2000; 10: 154-167*).*

In the case of nuts, the most representative bioactive compounds are:
- unsaturated fatty acids: monounsaturated, such as oleic, palmitoleic and gadoleic acids; and polyunsaturated, such as linoleic and linolenic acids,
- fiber, especially the soluble type,
- phytosterols, such as stigmasterol, campesterol and β-sitosterol,
- polyphenols, especially in skin,
- minerals, such as selenium, copper, magnesium, potassium, zinc and calcium,
- vitamin E,
- folic acid, and
- amino acids, such as arginine or lysine.

In the case of cocoa, its unsaturated fatty acid, fiber, phytosterol and polyphenol content must be emphasized (Watherhouse A, Shirley R, Donovan J. Antioxidants in chocolate. Lancet, 1996; 348:834*;* Kondo K, Hirano R, Matsumoto A, Igarashi O, Ikatura H. Inhibition of LDL oxidation by cocoa. Lancet, 1996, 348:1514*); (*Kris-Etherton PM, Keen CL. Evidence that the antioxidant flavonoids in tea and cocoa are beneficial for cardiovascular health. Current Opinion Lipidol 2002, 13:41-49*).*

The presence of all these bioactive components, some of which are already classified as functional components, is not only interesting on an individual level, but also considering their effect as a whole, i.e., analyzing the synergies that can exist among them, multiplying the beneficial health effect. For example, if these ingredients are mixed in a combined matrix, as is the case of nut and dried fruit creams for a confection, in which there could be a majority nut and dried fruit and cocoa content, the interaction among the bioactive components that come from the different raw materials also provide an additional positive effect in cascade *(*Wollgast J, Anklam E. Review on polyphenols in Theobroma cacao: changes in composition during the manufacture of chocolate and methodology for identification and quantification. Food Research International. 2000, 33: 423-447*), (*EP1106073*); (WO99*/*45797).*

There are thorough studies and patent documents relating to the preparation of functional foods incorporating some of the ingredients which are used in creams. One of these patents (*WO99*/*45797*) proposes preparing dark or milk chocolate, incorporating nuts, obtaining a product rich in polyphenols such as procyanidins and an amino acid such as L-arginine, with preventive effects in cardiovascular diseases and cancer. Following the same line, other studies evaluate the benefits of incorporating nuts in chocolates and sweets *(*Tikellis K. Nutrition and confectionery. Manufacturing confectioner, 81 (4) 87-90, 2001*; (*Sectzer JR. Sawier tastes, increased health benefits boost fruit and nut appeal. Candy Industry; 166 (5) 42-44, 2001*); (*Sectzer JR. Harvesting the full potential of nuts. Candy Industry; 167(1) 50-54, 2002). Another patent provides different formulations starting from cocoa and derivatives thereof for the purpose of seeking the synergistic effect between two or more components, one of such components always being polyphenols, specifically procyanidins (US6610320).

Processes have recently been patented which aim to minimize the losses of functional components. This is the case of a process (EP1106073) by means of which a cocoa powder rich in polyphenols is obtained. Another study, conducted by members of the DG Joint Research Center for Health & Consumer Protection, Food Products & Consumer Goods Unit of the European Union, evaluates the changes of the composition during the manufacture of chocolate and the methodology of analysis, isolation, purification and identification of the different components of interest (*Wollgast J. et al 2000*).

There is a wide variety of nuts available and commonly processed as raw materials in the preparation of the previously mentioned creams such as: hazelnut, almonds, walnuts and mixtures thereof. Each of them has their interest from the point of view of their composition in bioactive components. Out of the products derived from cocoa, cocoa butter and cocoa powder, as well as the extracts of their different fractions, and cocoa liquor, are used.

Accordingly, both types of foods are absolutely recommendable from the health point of view, in addition to their widely known attributes in relation to their enjoyable aspect and palatability. To that effect it is proven that the incorporation of products with considerable appeal from the organoleptic point of view makes it easier to incorporate them in the diet (Tecnifood, November 2002, 18-39*).*

However, in addition to the conventional ingredients in the formulation of creams, the authors of the present invention propose the use of alternative ingredients, of a preferably natural origin, which are interesting due to their high content in any of the functional components set forth.

Based on this the authors of the present invention have surprisingly found that through a composition which, in addition to containing the proposed mixtures of traditional ingredients (nuts and cocoa), contains certain alternative ingredients selected from the groups consisting of vegetable oils rich in unsaponifiable lipids and flours rich in soluble fiber (with water retention capacity), many of the active ingredients present in different food matrices act synergistically in the positive regulation of a certain physiological function and/or in reducing the risk of developing a certain vascular, mainly cardiovascular, disease.

The main inventive step of this invention is based on the balanced combination of food ingredients which had not been previously combined with one another and which, as a result of the natural allowance of active ingredients from each of them, provides a synergistic effect measurable in biomarkers and certain physiological functions which indicate the reduction of the risk of suffering a vascular disease, including both cardiovascular and neurodegenerative diseases, because they share common risk factors measurable by the same biomarkers and physiological functions.

In this sense, by means of the mentioned combination of ingredients it is able to act mainly on biomarkers such as LDL cholesterol and blood pressure which the scientific community *(*Aggett, P.J. et al., PASSCLAIMS. Process for the Assessment of Scientific Support for claims on Foods. Eur J. Nutr, 2005, 44:I/1-I/2*)* for now considers to be the only biomarkers that are methodologically valid and directly related to acting on the risk of suffering a cardiovascular disease.

Therefore the present invention enhances the combination of ingredients of a natural origin which, combined with one another, supply effective amounts of soluble fiber with water retention capacity, unsaturated fatty acids and unsaponifiable lipids. Said combination is proven to have a direct effect on the regulation of lipid profiles, and especially on the reduction of LDL and total cholesterol, as well as on the reduction of systolic and diastolic blood pressure.

This novel product is aimed at a broad sector of the population (all ages) who wish to prevent and reduce the risk of suffering cardiovascular diseases (and vascular diseases in general) and, in some cases, other short- and long-term degenerative diseases, without renouncing the pleasure of eating said product. Furthermore, the inclusion of these novel foods in the diet does not increase body weight.

What is therefore sought with the product of invention is to find new alternatives in incorporating foods rich in functional components, with a beneficial action on human health, into the diet. In summary, the invention attempts to diversify the manner of consuming these foods, preventing the monotony influencing in the abandonment of certain diets.

### Object of the Invention

The main objective of the present invention is to obtain a food formulation having a positive effect in the prevention of vascular diseases in mammals, including man.

It further contemplates the use of said food formulation in preparing a food and/or dietary product for the prevention of vascular diseases in mammals, including man.

Another object of the invention is a functional food obtained from said food formulation.

Finally, an object of the present invention is a method for preventing vascular diseases in mammals, including man, based on ingesting said food formulation.

### Description of the Invention

The authors of the present invention, which is defined by the claims, have focused on evaluating the effect of certain mixtures of ingredients in the prevention of diseases of a vascular origin (such as cardiovascular and neurodegenerative diseases), verifying the positive synergistic effect of the bioactive components supplied by the ingredients to the mixture on certain biomarkers and physiological responses related to said diseases.

Therefore, the present invention consists of obtaining a food formulation obtained from a complex and balanced mixture of effective amounts of at least one ingredient from each of the four groups consisting of nuts, cocoa, vegetable oils rich in unsaponifiable lipids and flours rich in soluble fiber, with water retention capacity, wherein said mixture provides effective minimum amounts of at least the following bioactive compounds: soluble fiber, unsaponifiable lipids and unsaturated fatty acids, the combination of which produces a positive effect in the prevention of vascular, preferably cardiovascular, diseases in mammals, including man.

Said complex and balanced mixture of ingredients is based on the combination and processing of effective amounts of different, preferably natural, raw materials which can supply to the mixture a minimum of different bioactive components acting synergistically and effectively, without being isolated from their original matrix, which allows other components of the ingredient to be maintained in the original matrix. The features of this mixture allow its inclusion in a balanced diet, further providing, as functional food, health benefits. Since it is a balanced matrix, there is no large excess of any of the active ingredients, but rather it is the combination of several of these ingredients which achieves, by means of synergies, the benefit in usual intake conditions in a balanced diet.

Therefore, the particularity of this novel food formulation is based on the fact that the mentioned synergistic effect is achieved in a single formulation/food/nutritional supplement/dietary supplement/mixture and in the usual intake proportions. Thus, by including it as food or part of a food in a balanced diet, its effect can be measured by comparing it with the effect produced by similar formulations from the nutritional and caloric point of view. The difference with other similar inventions/products of the state of the art consists of the features of the added ingredients from the point of view of their content in active ingredients and/or of the balance/combination between them. Unlike some foods or food ingredients, which consist of the simple addition of one or several active ingredient concentrates in a conventional food matrix, in this case it is the actual complex matrix which mainly or exclusively supplies said active ingredients and therefore the effect as a functional food is mainly or exclusively due to said matrix.

In a preferred embodiment, the soluble fiber content present in the formulation is at least 3-7%, the unsaponifiable lipid content is at least 0.2-1.6% and the unsaturated fatty acid content is at least 17-35%.

In particular embodiments of the invention, the unsaponifiable lipids are sterols and/or their esters, stanols and/or their esters, tocopherols, tocotrienols, oryzanol or their mixtures and the unsaturated fatty acids are monounsaturated, polyunsaturated or a mixture of both. In a preferred embodiment, the vegetable fat content is based on a balanced mixture of fatty acids, mainly monounsaturated and polyunsaturated fatty acids.

The ingredients which can be used in the preparation of the formulation/functional food, from the vascular benefit point of view, of the present invention, belong to the following groups of raw materials or fractions and/or extracts thereof:
- Nuts: Included within the range of nuts potentially usable in the formulation are: almonds, hazelnuts, walnuts, pecans, pistachios, pine nuts, cashews, Brazil nuts, macadamia nuts, peanuts, etc. Depending on their composition, nuts having a higher content in bioactive components, including the proportion in fatty acids (mainly unsaturated fatty acids) and specifically those representing higher activity in reducing the cardiovascular risk, are preferred. The present invention contemplates the use of both just one type of nut or fraction and of mixtures thereof.
- Cocoa: the choice of this ingredient also corresponds to its interesting composition in bioactive components with a cardiovascular health benefit. It can be used in its different forms: cocoa paste, cocoa liquor, cocoa butter and/or cocoa powder, and/or their respective fractions or extracts of the same.
- Vegetable oils rich in unsaponifiable lipids: contemplated in this category are vegetable oils with an interesting composition in bioactive components from the vascular health point of view, both in reference to their unsaturated fatty acid content and their content in unsaponifiables (phytosterols, tocopherols, tocotrienols,...). Some of these oils are nut oils, different seed oils (sunflower, linseed, sesame, pumpkin, rapeseed,...), rice bran and/or germ oils, rich in gamma oryzanol, wheat oil, corn oil and oils from other grains, oils from other sources (algae, soy, safflower, evening primrose, "tall oil" from wood, or other oils that may later appear). Also included in this group are the fractions or extracts of these ingredients rich in said bioactive components.
- Flours (fractions of ground vegetables and/or powdery substances or powder substances) with a significant soluble fiber content: mainly soluble type vegetables with a high fiber content are selected, but a high insoluble fiber content is not discarded, while at the same time priority is given to the choice of those vegetables further having a composition also rich in other bioactive compounds with regard to cardiovascular benefit, without neglecting at any time the organoleptic profile of the mixture. Some of these vegetable flours comprise: Plantago ovata flour, flours rich in beta glucans such as oat flour or fractions or concentrates obtained therefrom, barley flour or fractions or concentrates obtained therefrom, mushroom flour, such as reishi mushroom flour or fractions or concentrates obtained therefrom, carob bean flour and fruit extracts/fractions rich in pectins, cellulose derivatives or their mixtures. The interest of incorporating carob bean flour is mainly due to its high fiber and antioxidant content, as well as its sweet flavor, which are useful aspects in the formulation.

This group of ingredients provides a soluble fiber allowance that can retain considerable water content. Therefore due to the viscous fluid that is generated in the digestive apparatus, the cholesterol content, which is later present in plasma, can be reduced. They simultaneously can further supply other components of interest such as insoluble fiber, antioxidants, etc...

Besides containing active ingredients that can act in the positive regulation of a certain physiological function and/or in reducing the risk of developing a vascular disease, the mentioned ingredients can also have a positive function in the prevention of degenerative-type diseases such as neurodegenerative diseases or cancer.

In addition, if necessary due to organoleptic, technological, functional or other characteristics, in a particular embodiment of the invention the use of the following ingredients in the composition of the formulation is also contemplated:
- Reinforcement components of the formulation of mixtures rich in bioactive components: this type of ingredient comprises compounds which are normally used in the formulation of creams such as: lecithin (as an emulsifier), inulin (which reduces the glycemic index and provides other benefits such as the prebiotic benefit), tocopherols (as antioxidants of a natural origin) or other unsaponifiable lipid concentrates or, in some cases, folic acid (to reinforce the natural content of nuts, the content of which can be reduced after they are processed), or others.
- Complementary components: Sugars and/or sweeteners and/or other condiments (salt, pepper, spices). This type of ingredient corresponds to organoleptic needs of the finished product. Although there is a wide range of sugars and sweeteners available, the criterion of choice is the minimum addition amount with which a satisfactory organoleptic profile is obtained and/or the glycemic index is reduced as much as possible. Examples of sweeteners include: freeze-dried fruit syrups, freeze-dried grape must, freeze-dried honey, others.
   The addition or not of this type of ingredient basically corresponds to the criteria of the final application that will be given to the functional mixture. In the case of a sauce for cooking, the sugar allowance can be substituted with a small allowance of salt or another condiment or seasoning, without losing the organoleptic characteristics of the product.

In a preferred embodiment, the food formulation can comprise the following additional bioactive compounds: polyphenols, folic acid, arginine, lysine and selenium, all of which are compounds that can be found or can be provided by any of the four mentioned groups of ingredients (cocoa, nuts, flours and vegetable oils) or by any of the other reinforcement or complementary ingredients.

In a particular embodiment of the invention, the vegetable oil used in the composition of the formulation is rice bran and/or germ oil, of the vegetable oil group, preferably with a percentage of at least 1% of gamma oryzanol.

In another particular embodiment of the invention, the flour used in the formulation is selected from Plantago ovata flour, oat flour, fractions of oat flour rich in beta glucan, barley flour, fractions of barley flour rich in beta glucan, reishi mushroom flour, fractions of reishi mushroom flour rich in beta glucan, vegetable fractions rich in it, fruit extracts/fractions rich in pectins, carob bean flour, cellulose derivatives or their mixtures.

The food formulations contemplated in the present invention can be taken in individually as food or incorporated as an ingredient in another composite food, being able to be incorporated in a balanced manner in a healthy diet providing additional health benefits to said diet.

In a preferred embodiment of the invention, the formulation is integrated in a solution and/or dispersion and/or balanced mixture of fats of a natural vegetable origin.

Comprised in this concept, one of the particular embodiments contemplated in the present invention is a food based on a balanced mixture of raw materials (such as nuts, cocoa, vegetable oils rich in unsaponifiable lipids), with a predominance of unsaturated fats, and other raw materials (such as vegetable flours or fruit extracts/fractions rich in pectins, all of which are rich in soluble fiber with water retention capacity) in the form of powder, particles in suspension or paste which can be dispersed in the formed fat matrix.

In preferred embodiments, the obtained mixture is fluid and homogeneous which, for a certain composition, facilitates bioaccessibility and subsequent bioavailability of the bioactive compounds.

Accordingly, the resulting food consists of a fat matrix in which a certain amount of solid particles, which can come from both the materials with a fatty base and from others in which their fat content is much lower or inexistent, is dispersed. Most of these raw materials supply a content of active ingredients (phytosterols, unsaturated fatty acids, soluble fiber, polyphenols, tocopherols, tocotrienols,...) of a natural origin which by themselves in each isolated raw material produce a lower effect on one or several certain biomarkers indicating a reduction of the risk of suffering a certain disease. Furthermore, in some cases the intake of some of them separately is not plausible or possible given the organoleptic characteristics of the product (for example carob bean or Plantago ovata flour).

Therefore in a particular embodiment, a food formulation is contemplated in which the natural ingredients used in its composition are integrated in said solution and/or dispersion and/or balanced mixture of vegetable fats in the form of particles in suspension. Said formulation can additionally incorporate inclusions of cookies, grains, textured grains, dried fruits or freeze-dried fruits.

The concentration of each of the bioactive compounds in the different ingredients selected can in some cases be individually insufficient to produce a significant effect on the prevention of the vascular risk, specifically on the factors or indicators that the scientific community accepts as having a direct relationship with the vascular system, and in particular with cardiovascular diseases (cholesterol, LDL cholesterol, blood pressure (diastolic, systolic), waist girth, apo B/apo A-I ratio, inflammation and oxidation biomarkers).

Based on this aspect, in the formulation of the product of the invention the concentrations of all the compounds considered to be of interest in relation to the beneficial vascular effects are accounted for. The compounds considered to be of interest are those which are currently considered to be functional (fiber, specifically soluble fiber, phytosterols, polyphenols, linoleic acid and linolenic acid) and others for which there is scientific evidence in epidemiological and intervention studies of their efficacy in reducing cardiovascular risk (folic acid, selenium, vitamin E, arginine and lysine, others).

For the purpose of being able to concentrate the content in bioactive compound if needed, different techniques are proposed for concentrating said compound in the same matrix, such as concentration by drying and crushing and obtaining extracts, selective fractioning, etc, as well as the possible encapsulation or protection of some of the ingredients, for the purpose of eliminating unwanted flavors and aromas and protecting those which are sensitive to different processing operations.

As previously mentioned the food formulation of the invention has measurable positive effects on specific biomarkers and/or physiological functions relating to vascular, preferably cardiovascular, disease.

Therefore in a particular embodiment, the formulation provides a measurable synergistic effect on the reduction of total cholesterol and/or LDL cholesterol in mammals, including man.

Particularly, a formulation based on ingredients of a vegetable origin, which contain at least a minimum amount of each of the following first three groups of active ingredients and which are suitably mixed, provide a hypocholesterolemic effect:
- unsaponifiable lipids
- soluble fiber
- unsaturated fatty acids
- polyphenols
- folic acid

In another preferred embodiment, the formulation of the present invention provides a measurable positive synergistic effect on the reduction of blood pressure (systolic and diastolic) in mammals, including man.

In another preferred embodiment, the formulation causes a reduction of the apo B/apo A-I ratio.

Furthermore, the inclusion of this formulation in the diet does not involve an increase in body weight in humans due to the fact that the nutrient and caloric allowance is balanced. In the same manner, in particular embodiments of the invention, the intake of the formulation may involve a waist girth reduction.

This invention comprises the formulation of a novel product concept consisting of a matrix based on a balance of vegetable fats of a natural origin, with a predominance of unsaturated fats, and a group of natural ingredients selected for their composition rich in bioactive compounds and for their synergistic benefits with regard to health, enhancing at all times the organoleptic characteristics that are pleasing for consumers, providing an organoleptic effect that is similar to other products of a similar organoleptic profile but which do not have beneficial health effects or which are even harmful due to their unbalanced composition.

The benefit produced by the formulation of the invention on health occurs in normal intake conditions, this point being taken into account both at the time of formulating said product and after the processing thereof. Therefore the benefit takes place even after the formulation is processed to obtain the preparation of the end product, and even after being stored.

The formulations comprised in the present invention can be used for the preparation of quality confectionery products, ice-cream products, sauces, dietary products, and can even be included in meat matrices, soy derivative matrices (such as tofu), in fresh stuffed pasta, etc. given their highly pleasing organoleptic profile without sacrificing at any time their healthy aspect with regard to the risk of suffering vascular diseases.

Therefore in another aspect of the invention, the use of the formulation in preparing a food and/or dietary product having positive effects in the prevention of vascular diseases in mammals, including man, is contemplated.

Another aspect of the invention relates to a functional food comprising the food formulation described in the present invention. Once processed, said functional food preserves the previously described health benefit properties. In preferred embodiments of the invention, the actual food formulation object of the invention is by itself a finished functional food.

Finally, another aspect of the invention contemplates the method for preventing vascular diseases in mammals, preferably man, based on the intake of the food formulation described in the present invention.

Featured below by way of example is the following formulation obtained (cream A), not to be considered as limiting or restrictive of the present invention, including the following ingredients:

### Example 1

| **Ingredient** | **Percentage %** |
|---|---|
| Hazelnut paste | 38.0 |
| Cocoa liquor | 11.2 |
| Cocoa butter | 3.8 |
| Rice bran oil | 12.5 |
| Carob bean flour | 7.0 |
| Plantago ovata flour | 6.5 |
| Sucrose | 12.8 |
| Fructose | 8.0 |
| Encapsulated folic acid | 0.0024 |
| Tocopherols | 0.040 |
| Lecithin | 0.30 |

These ingredients were refined and mixed in equipment commonly used in the manufacture of creams with cocoa and nuts in pilot and industrial processes, producing a homogenous mixture formed by a dispersion of the refined solid particles, with a particle size in the order of 30 □m, in the balanced mixture of vegetable fats.

By means of an intervention study, this cream incorporated in individual doses in a balanced diet was compared with two types of cream:
a) standard or control cream, made up of cocoa powder (7.0%), sunflower oil (33.2%), palm oil (11.8%), sucrose (47.6%), tocopherols (0.040%) and lecithin (0.30%).
b) cream B, made up of hazelnut paste (36.4%), butter (3.6%) and cocoa liquor (11.7%), sunflower oil (9.6%), palm oil (1.8%), phytosterols of natural sources of a vegetable origin (4.1%), sucrose (32.7%), tocopherols (0.040%) and lecithin (0.30%).

The intervention study lasted for six weeks. In the first two weeks, all the individuals involved consumed six daily doses (of 13 g each) of the control cream. In the following four weeks, the individuals were distributed into different groups consuming one of the different mentioned confections or creams. The daily consumed doses of each of the creams in this period were the same as during the first two weeks.

When the study ended, a positive effect of formulation A on the markers of the risk of vascular disease (cholesterol, LDL cholesterol, waist girth, Apo B/Apo A-I), as well as on physiological function (reduction of blood pressure), was observed. It could specifically be observed that the reduction of the total and LDL cholesterol concentrations in plasma of the participants was significant with regard to the starting values in those who consumed creams of one of the two novel formulations (A and B). In the case of the standard formulation, said total cholesterol and LDL cholesterol levels experienced no significant changes.

The formulation mentioned first, formed by a more complex and balanced mixture of natural ingredients rich in active ingredients (formulation or cream A), caused a greater reduction of these indicators. In this case the reduction of total cholesterol was 9% (variation from baseline values) whereas the reduction of LDL cholesterol was 10% (variation from baseline values). In the case of cream B, the reduction was 9 and 12%, respectively. Based on these results it was proven that the use of lower allowances of each of the active ingredients (taking into account that they have not been isolated from the matrix) combined with one another yield results that are comparable to the direct addition of isolated phytosterols used in B.

It was also determined that cream A caused a significant 7% reduction of the apo B/apo A-I ratio.

For cream A, the reduction of blood pressure (variation from baseline values) was also significant (3% for systolic blood pressure and 3% for diastolic blood pressure). Furthermore, in the case of cream A, a significant waist girth reduction (-1.4 cm) was observed

The body weight variation due to the fact of incorporating creams A or B in the diet according to the described methodology was nil (in other words, it was not significant).

95% confidence intervals were used to determine whether or not there were significant variations.

### Example 2

As in the previous case for a particular application this mixture was mixed by means of industrial refining and mixing equipment which allowed obtaining a homogenous dispersion of solid particles of about 30 µm in size in a balanced mixture of vegetable fats (mainly formed by unsaturated fatty acids). The different ingredients are thus more bioaccessible.

The obtained formulation was:

| **Ingredient** | **Percentage %** |
|---|---|
| Almond | 35 |
| Cocoa powder | 7 |
| Cocoa butter | 12 |
| Rice germ oil | 15 |
| Apple flour rich in pectins | 10 |
| Inulin | 10 |
| Sucrose | 11 |
| Tocopherols | 0.040 |
| Lecithin | 0.30 |

### Example 3

| **Ingredient** | **Percentage %** |
|---|---|
| Walnuts | 20 |
| Almond | 15 |
| Cocoa liquor | 20 |
| Corn oil | 5 |
| 30% oryzanol rice bran oil | 5 |
| Oat flour rich in beta glucan | 12 |
| Inulin | 12 |
| Fructose | 11 |
| Folic acid | 0.0024 |
| Tocopherols | 0.040 |
| Lecithin | 0.30 |

## Claims

1. A food formulation **characterized in that** it comprises a mixture of effective amounts of at least one ingredient from each of the four groups consisting of nuts, cocoa, vegetable oils rich in unsaponifiable lipids and flours rich in soluble fiber with water retention capacity, wherein said mixture provides at least the following bioactive compounds: soluble fiber in an effective minimum amount of at least 3%, unsaponifiable lipids in an effective minimum amount of at least 0.2%, and unsaturated fatty acids in an effective minimum amount of at least 17%.

2. A food formulation, according to claim 1, **characterized in that** the mixture provides at least the following bioactive compounds: soluble fiber in an effective minimum amount comprised between 3-7%, unsaponifiable lipids in an effective minimum amount comprised between 0.2-1,6%, and unsaturated fatty acids in an effective minimum amount comprised between 17-35%.

3. The food formulation according to any of the previous claims, **characterized in that** the ingredients are of a natural origin.

4. The food formulation according to claim 1, **characterized in that** the unsaponifiable lipids are sterols and/or their esters, stanols and/or their esters, tocopherols, tocotrienols, oryzanol or their mixtures and the unsaturated fatty acids are monounsaturated, polyunsaturated or a mixture of both.

5. The food formulation according to any of the previous claims, **characterized in that** it additionally comprises other components selected from reinforcement components of the formulation of mixtures rich in bioactive components, sugars, sweeteners, other condiments and their mixtures.

6. The food formulation according to any of the previous claims, **characterized in that** it additionally comprises polyphenols, folic acid, arginine, lysine and selenium.

7. The food formulation according to any of the previous claims, **characterized in that** one of the ingredients used in its composition is rice bran and/or germ oil.

8. The formulation according to claim 7, **characterized in that** the rice bran and/or germ oil is rich by at least 1% in gamma oryzanol.

9. The formulation according to any of the previous claims, **characterized in that** one of the ingredients used in its composition is a flour selected from Plantago ovata flour, oat flour, fractions of oat flour rich in beta glucan, barley flour, fractions of barley flour rich in beta glucan, reishi mushroom flour, fractions of reishi mushroom flour rich in beta glucan, carob bean flour, vegetable fractions rich in it, fruit extracts/fractions rich in pectins, cellulose derivatives or their mixtures.

10. The food formulation according to any of the previous claims, **characterized in that** the ingredients used in its composition form a homogenous dispersion of solid particles integrated in a balanced mixture of fats of a vegetable origin.

11. The food formulation according to claim 10, **characterized in that** it additionally comprises inclusions selected from cookies, grains, textured grains, dried fruits and freeze-dried fruits.

12. Food formulation, according to any of claims 1-11, for use in the prevention of vascular diseases in mammals, including man.

13. Food formulation, according to claim 12, wherein the vascular diseases are cardiovascular diseases.

14. Food formulation, according to claim 12, wherein the vascular diseases are neurodegenerative diseases.

15. Food formulation, according to claim 12, wherein the prevention is carried out through the reduction of total cholesterol and LDL cholesterol in mammals, including man.

16. Food formulation, according to claim 12, wherein the prevention is carried out through the reduction of blood pressure in mammals, including man.

17. Food formulation, according to claim 12, wherein the prevention is carried out through the reduction of the Apo B/Apo A-I ratio in mammals, including man.

18. Use of the food formulation according to claims 1-17 in preparing a food and/or dietary product for use in the prevention of vascular diseases in mammals, including man.

19. A functional food comprising a food formulation according to claims 1-17.

## Patentansprüche

1. Nahrungszubereitung, **dadurch gekennzeichnet, dass** sie ein Gemisch aus wirksamen Mengen wenigstens eines Bestandteils aus jeder der vier Gruppen umfasst, die aus Nüssen, Kakao, Pflanzenölen, die reich an nicht verseifbaren Lipiden sind, und Mehlen, die reich an löslichen Fasern mit Wasserrückhaltekapazität sind, bestehen, wobei das Gemisch wenigstens die folgenden bioaktiven Verbindungen bereitstellt: lösliche Faser in einer wirksamen minimalen Menge von wenigstens 3 %, nicht verseifbare Lipide in einer wirksamen minimalen Menge von wenigstens 0,2 % und ungesättigte Fettsäuren in einer wirksamen minimalen Menge von wenigstens 17 %.

2. Nahrungszubereitung gemäß Anspruch 1, **dadurch gekennzeichnet, dass** das Gemisch wenigstens die folgenden bioaktiven Verbindungen bereitstellt: lösliche Faser in einer wirksamen minimalen Menge von 3 - 7 %, nicht verseifbare Lipide in einer wirksamen minimalen Menge von 0,2 - 1,6 % und ungesättigte Fettsäuren in einer wirksamen minimalen Menge von 17 - 35 %.

3. Nahrungszubereitung gemäß einem der vorstehenden Ansprüche, **dadurch gekennzeichnet, dass** die Bestandteile natürlichen Ursprungs sind.

4. Nahrungszubereitung gemäß Anspruch 1, **dadurch gekennzeichnet, dass** die nicht verseifbaren Lipide Sterole und/oder ihre Ester, Stanole und/oder ihre Ester, Tocopherole, Tocotrienole, Oryzanol oder deren Gemische sind und die ungesättigten Fettsäuren einfach ungesättigt, mehrfach ungesättigt oder ein Gemisch von beiden sind.

5. Nahrungszubereitung gemäß einem der vorstehenden Ansprüche, **dadurch gekennzeichnet, dass** sie zusätzlich noch andere Komponenten umfasst, die aus Verstärkungskomponenten der Zubereitung an Gemischen, die reich an bioaktiven Komponenten sind, Zuckern, Süßstoffen, anderen Gewürzen und ihren Gemischen ausgewählt sind.

6. Nahrungszubereitung gemäß einem der vorstehenden Ansprüche, **dadurch gekennzeichnet, dass** sie zusätzlich noch Polyphenole, Folsäure, Arginin, Lysin und Selen umfasst.

7. Nahrungszubereitung gemäß einem der vorstehenden Ansprüche, **dadurch gekennzeichnet, dass** einer der in der Zusammensetzung verwendeten Bestandteile Reiskleie- und/oder -keimöl ist.

8. Zubereitung gemäß Anspruch 7, **dadurch gekennzeichnet, dass** das Reiskleie- und/oder -keimöl um wenigstens 1 % reich an Gamma-Oryzanol ist.

9. Zubereitung gemäß einem der vorstehenden Ansprüche, **dadurch gekennzeichnet, dass** einer der in der Zusammensetzung verwendeten Bestandteile ein Mehl ist, das aus Flohsamenpulver, Hafermehl, an beta-Glucan reichen Fraktionen von Hafermehl, Gerstenmehl, an beta-Glucan reichen Fraktionen von Gerstenmehl, Reishi-Pulver, an beta-Glucan reichen Fraktionen von Reishi-Pulver, Johannisbrotkernmehl, Pflanzenfraktionen, die reich daran sind, an Pektinen reichen Fruchtextrakten/Fraktionen, Cellulosederivaten oder ihren Gemischen ausgewählt ist.

10. Nahrungszubereitung gemäß einem der vorstehenden Ansprüche, **dadurch gekennzeichnet, dass** die in der Zusammensetzung verwendeten Bestandteile eine homogene Dispersion von festen Teilchen bilden, die in einem ausgewogenen Gemisch von Fetten pflanzlichen Ursprungs integriert sind.

11. Nahrungszubereitung gemäß Anspruch 10, **dadurch gekennzeichnet, dass** sie zusätzlich Einschlüsse umfasst, die aus Keksen, Körnern, texturierten Körnern, getrockneten Früchten und gefriergetrockneten Früchten ausgewählt sind.

12. Nahrungszubereitung gemäß einem der Ansprüche 1 bis 11 zur Verwendung bei der Prävention von Gefäßkrankheiten bei Säugern einschließlich des Menschen.

13. Nahrungszubereitung gemäß Anspruch 12, wobei die Gefäßkrankheiten Herz-Kreislauf-Erkrankungen sind.

14. Nahrungszubereitung gemäß Anspruch 12, wobei die Gefäßkrankheiten neurodegenerative Erkrankungen sind.

15. Nahrungszubereitung gemäß Anspruch 12, wobei die Prävention durch die Reduktion von Gesamtcholesterin und LDL-Cholesterin bei Säugern einschließlich des Menschen durchgeführt wird.

16. Nahrungszubereitung gemäß Anspruch 12, wobei die Prävention durch die Reduktion des Blutdrucks bei Säugern einschließlich des Menschen durchgeführt wird.

17. Nahrungszubereitung gemäß Anspruch 12, wobei die Prävention durch die Reduktion des Apo-B/Apo-A-I-Verhältnisses bei Säugern einschließlich des Menschen durchgeführt wird.

18. Verwendung der Nahrungszubereitung gemäß den Ansprüchen 1 - 17 bei der Herstellung eines Nahrungsmittels und/oder diätetischen Lebensmittels zur Verwendung bei der Prävention von Gefäßkrankheiten bei Säugern einschließlich des Menschen.

19. Funktionelles Lebensmittel, umfassend eine Nahrungszubereitung gemäß den Ansprüchen 1 - 17.

## Revendications

1. Formulation alimentaire **caractérisée en ce qu'**elle comprend un mélange de quantités efficaces d'au moins un ingrédient de chacun des quatre groupes constitués de noix, de cacao, d'huiles végétales riches en lipides insaponifiables et de farines riches en fibres solubles ayant une capacité de rétention d'eau, ledit mélange comprenant au moins les composés bioactifs suivants : des fibres solubles en une quantité minimale efficace d'au moins 3 %, des lipides insaponifiables en une quantité minimale efficace d'au moins 0,2 %, et des acides gras insaturés en une quantité minimale efficace d'au moins 17 %.

2. Formulation alimentaire selon la revendication 1, **caractérisée en ce que** le mélange comprend au moins les composés bioactifs suivants : des fibres solubles en une quantité minimale efficace comprise entre 3 et 7 %, des lipides insaponifiables en une quantité minimale efficace comprise entre 0,2 et 1,6 %, et des acides gras insaturés en une quantité minimale efficace comprise entre 17 et 35 %.

3. Formulation alimentaire selon l'une quelconque des revendications précédentes, **caractérisée en ce que** les ingrédients sont d'origine naturelle.

4. Formulation alimentaire selon la revendication 1, **caractérisée en ce que** les lipides insaponifiables sont des stérols et/ou leurs esters, des stanols et/ou leurs esters, des tocophérols, des tocotriénols, l'oryzanol ou leurs mélanges, et les acides gras insaturés sont monoinsaturés, polyinsaturés ou un mélange des deux.

5. Formulation alimentaire selon l'une quelconque des revendications précédentes, **caractérisée en ce qu'**elle comprend en outre d'autres composants choisis parmi des composants de renforcement de la formulation de mélanges riches en composants bioactifs, des glucides, des édulcorants, d'autres condiments et leurs mélanges.

6. Formulation alimentaire selon l'une quelconque des revendications précédentes, **caractérisée en ce qu'**elle comprend en outre des polyphénols, de l'acide folique, de l'arginine, de la lysine et du sélénium.

7. Formulation alimentaire selon l'une quelconque des revendications précédentes, **caractérisée en ce qu'**un des ingrédients utilisés dans sa composition est constitué d'huile de son de riz et/ou d'huile de germe de riz.

8. Formulation selon la revendication 7, **caractérisée en ce que** l'huile de son de riz et/ou l'huile de germe de riz sont riches d'au moins 1 % en gamma-oryzanol.

9. Formulation selon l'une quelconque des revendications précédentes, **caractérisée en ce qu'**un des ingrédients utilisés dans sa composition est une farine choisie parmi la farine de Plantago ovata, la farine d'avoine, des fractions de farine d'avoine riches en bêta-glucane, la farine d'orge, des fractions de farine d'orge riches en bêta-glucane, la farine de champignon Reishi, des fractions de champignon Reishi riches en bêta-glucane, la farine de caroube, des fractions végétales riches en celle-ci, des extraits/fractions de fruits riches en pectines, des dérivés de cellulose ou leurs mélanges.

10. Formulation alimentaire selon l'une quelconque des revendications précédentes, **caractérisée en ce que** les ingrédients utilisés dans sa composition forment une dispersion homogène de particules solides intégrées dans un mélange équilibré de graisses d'origine végétale.

11. Formulation alimentaire selon la revendication 10, **caractérisée en ce qu'**elle comprend en outre des inclusions choisies parmi des biscuits, des grains, des grains texturés, des fruits secs et des fruits lyophilisés.

12. Formulation alimentaire selon l'une quelconque des revendications 1 à 11, destinée à être utilisée dans la prévention de maladies vasculaires chez les mammifères, y compris l'homme.

13. Formulation alimentaire selon la revendication 12, dans laquelle les maladies vasculaires sont des maladies cardiovasculaires.

14. Formulation alimentaire selon la revendication 12, dans laquelle les maladies vasculaires sont des maladies neurodégénératives.

15. Formulation alimentaire selon la revendication 12, dans laquelle la prévention est effectuée par la réduction du cholestérol total et du cholestérol LDL chez les mammifères, y compris l'homme.

16. Formulation alimentaire selon la revendication 12, dans laquelle la prévention est effectuée par la réduction de la pression artérielle chez les mammifères, y compris l'homme.

17. Formulation alimentaire selon la revendication 12, dans laquelle la prévention est effectuée par la réduction du rapport Apo B/Apo A-I chez les mammifères, y compris l'homme.

18. Utilisation de la formulation alimentaire selon les revendications 1 à 17, dans la préparation d'un produit alimentaire et/ou diététique destiné à être utilisé dans la prévention de maladies vasculaires chez les mammifères, y compris l'homme.

19. Aliment fonctionnel comprenant une formulation alimentaire selon les revendications 1 à 17.
